# EUROPÄISCHE PATENTSCHRIFT

(11) **EP 3 416 698 B1**
(45) Veröffentlichungstag und Bekanntmachung des Hinweises auf die Patenterteilung: **12.10.2022**
(21) Anmeldenummer: 17705798.1
(22) Anmeldetag: 16.02.2017
(51) Int. Cl.: A61M 1/14, A61M 5/14, A61M 1/16

(54) **MEDIZINISCHES GERÄT UND VERFAHREN ZUM BETREIBEN EINES MEDIZINISCHEN GERÄTES**
MEDICAL DEVICE AND METHOD FOR OPERATING A MEDICAL DEVICE
APPAREIL MÉDICAL ET PROCÉDÉ POUR FAIRE FONCTIONNER UN APPAREIL MÉDICAL

(30) Priorität: 17.02.2016 DE 102016001868
(43) Veröffentlichungstag der Anmeldung: 26.12.2018
(73) Patentinhaber: Fresenius Medical Care Deutschland GmbH, 61352 Bad Homburg (DE)
(72) Erfinder: OFFERMANNS, Lars, 35510 Butzbach (DE)
(74) Vertreter: Herrmann, Uwe
(86) Internationale Anmeldenummer: PCT/EP2017/000228
(87) Internationale Veröffentlichungsnummer: WO 2017/140431

(56) Entgegenhaltungen:
- EP-A1- 1 872 814
- WO-A1-2012/113858
- US-A1- 2015 025 449
- US-A1- 2016 022 893
- US-B1- 6 447 492

## Beschreibung

Die vorliegende Erfindung betrifft ein medizinisches Gerät, insbesondere ein Blutbehandlungsgerät mit einer oder mehreren funktionalen Einheiten, die im Betrieb des Gerätes mit einem oder mehreren Disposables interagieren, wobei das medizinische Gerät des Weiteren ein oder mehrere Leuchtmittel aufweist und wobei das Gerät in unterschiedlichen Behandlungsmodi betreibbar ist.

Aus dem Stand der Technik ist es bekannt, Dialysegeräte mit Waagschalen auszuführen, in die im Rahmen der Vorbereitung des Gerätes zur Durchführung einer Dialysebehandlung Lösungsbeutel eingelegt werden. Die Lösungsbeutel verbleiben während der Dialysebehandlung in den Waagschalen. Bei den Lösungsbeuteln handelt es sich beispielsweise um Dialysat und/oder um Substituat enthaltende Beutel. Dabei ist es für den Behandlungserfolg sowie für die Patientensicherheit wesentlich, dass der richtige Lösungsbeutel in die richtige, d.h. für diesen vorgesehene Waagschale eingelegt wird.

Aus der DE 10 2013 008 213 A1 ist ein medizinisches Gerät bekannt, bei dem ein Teil Gerätes, wie beispielsweise das Gerätegehäuse beleuchtet wird, wodurch bestimmte Informationen, wie z.B. Füllstände oder Behandlungszeiten etc. wiedergegeben werden können.

Zudem offenbart die WO2012/113858 A1 eine Vorrichtung zum Unterstützen eines Bedieners bei der Bedienung eines medizinischen Geräts, umfassend eine Signalisiervorrichtung zum Signalisieren einer erforderlichen Bedienung einer zu bedienenden Komponente des medizinischen Geräts, bevorzugt durch Aussenden eines Signals von der zu bedienenden Komponente aus und/oder durch Richten eines Signals auf die zu bedienende Komponente.

Weiterhin offenbart US2016022893 extrakorporale Blutbehandlungsvorrichtungen mit Reservoirstatusleuchten und ein Verfahren zum Überwachen des Reservoirstatus unter Verwendung derselben.

Darüberhinaus offenbart EP1872814 A1 ein medizinisches System zur Abgabe und / oder Entnahme von Flüssigkeit an und / oder von einem Patienten.

Gemäß US2015025449 werden bei einer Blutbehandlungsmaschine verschiedene Alarmsignale ausgegeben. Aus der US6447492 ist zudem eine Drainagevorrichtung bekannt.

Der vorliegenden Erfindung liegt die Aufgabe zugrunde, ein medizinisches Geräte der eingangs genannten Art dahingehend weiterzubilden, dass die Wahrscheinlichkeit für eine fehlerhafte Zuordnung eines Disposables zu einer funktionalen Einheit gegenüber aus dem Stand der Technik bekannten Anordnungen verringert ist.

Diese Aufgabe wird durch ein medizinisches Gerät mit den Merkmalen des Anspruchs 1 gelöst. Danach ist vorgesehen, dass das Gerät über wenigstens eine Steuer- oder Regelungseinheit verfügt, die so ausgebildet sind, dass diese die Leuchtmittel in Abhängigkeit von dem ausgewählten Behandlungsmodus ansteuert, in dem das medizinische Gerät betrieben wird, wobei die Leuchtmittel derart angeordnet sind, dass das von diesen ausgehende Licht zumindest teilweise die wenigstens eine funktionale Einheit beleuchtet. Erfindungsgemäß handelt es sich bei der oder den funktionalen Einheiten um eine oder mehrere Waagschalen zur Aufnahme von zu wiegenden Lösungsbeuteln.

Die Leuchtmittel werden somit derart angesteuert, dass wenigstens eine Eigenschaft des von diesen ausgehenden Lichts von dem gewählten Behandlungsmodus abhängt.

So ist es beispielweise möglich, eine funktionale Einheit mit einer bestimmten Beleuchtungsfarbe zu beleuchten und eine andere funktionale Einheit mit einer bestimmten davon abweichenden Beleuchtungsfarbe.

So kann beispielsweise die Waagschale für den Lösungsbeutel, der das Dialysat enthält, in gelber Farbe angeleuchtet werden und die Waagschale für den Lösungsbeutel, der die Substitutionslösung enthält, in blauer Farbe.

Vorzugsweise entsprechen die Farben, in denen die eine oder mehreren funktionalen Einheiten beleuchtet werden, den Farben bzw. Farbcodierungen der Disposables, die mit den funktionalen Einheiten interagieren sollen, d.h. beispielsweise den auf Lösungsbeuteln angebrachten farblichen Codierungen.

Von der Erfindung ist jede beliebige von dem Behandlungsmodus abhängige Eigenschaft des Lichts umfasst, mit dem die wenigstens eine funktionale Einheit beleuchtet wird.

Vorzugsweise handelt es sich um die Farbe des Lichtes, von der Erfindung ist jedoch auch die Helligkeit, die Farbtiefe, der Kontrast, die Art der Beleuchtung, z;B. durchgehend, intermittierend etc. umfasst.

Bei der oder den funktionalen Einheiten handelt es sich um eine oder mehrere Waagschalen zur Aufnahme von zu wiegenden Lösungsbeuteln.

Vorzugsweise handelt es sich bei dem medizinischen Gerät um ein Dialysegerät. Von der Erfindung sind jedoch auch beliebige andere medizinische Geräte umfasst, bei denen Disposables, d.h. Einmalartikel zum Einsatz kommen, um eine Behandlung durchzuführen.

Wie oben ausgeführt, handelt es sich bei der oder den funktionalen Einheiten um eine oder mehrere Waagschalen zur Aufnahme von Lösungsbeuteln, deren Gewicht im Laufe der Behandlung durch eine mit der Waagschale in Verbindung stehende Wägezelle ermittelt wird.

Bei der oder den funktionalen Einheiten kann es sich beispielsweise um eine oder mehrere Halterungen oder Aufnahmen für ein oder mehrere Schlauchsets, Filter oder Dialysatoren handeln.

Eine besonders gute Erkennbarkeit ergibt sich, wenn die wenigstens eine funktionale Einheit teilweise oder vollständig transparent oder transluzent ausgebildet ist. Dies ermöglicht es beispielsweise im Falle von Waagschalen diese von unten zu beleuchten.

Grundsätzlich ist eine Beleuchtung der funktionalen Einheit von jeder beliebigen Seite denkbar und von der Erfindung mit umfasst.

Der Begriff "Beleuchtung" umfasst nicht nur den Fall, dass das Licht auf die funktionale Einheit fällt, sondern auch den Fall, dass das Licht von der funktionalen Einheit ausgeht, indem die Leuchtmittel beispielsweise im Inneren der funktionalen Einheit angeordnet sind oder indem beispielsweise eine flächige LED bzw. OLED auf die funktionale Einheit aufgebracht wird.

Eine weitere Beleuchtungsmöglichkeit besteht beispielsweise darin, das Licht ausgehend von einer Seitenkante der funktionalen Einheit in diese einzukoppeln, wodurch sich eine gute Erkennbarkeit und ein ansprechender optischer Gesamteindruck ergibt.

Die Art der Beleuchtung ist beliebig, solange sie für einen Nutzer gut erkennbar ist. Die Beleuchtung kann die gesamte funktionale Einheit oder nur einen Teil von dieser betreffen. Diese kann beispielsweise als gleichmäßige Beleuchtung ausgeführt sein oder auch eine bestimmt Form, beispielsweise in Form eines Schriftzuges oder eines Logos aufweisen.

Wie oben ausgeführt, können die Leuchtmittel derart ausgebildet sein, dass sie Licht beispielsweise in unterschiedlichen Farben, Helligkeiten oder in unterschiedlichen zeitlichen Abläufen abgeben.

Bei den Leuchtmitteln kann es sich beispielsweise um LEDs, insbesondere um RGB-LEDs handeln, so dass unterschiedliche Beleuchtungsfarben erzeugt werden können. Jedoch sind von der Erfindung auch beliebige andere Leuchtmittel, wie beispielweise Glühbirnen etc. umfasst.

In einer bevorzugten Ausgestaltung der Erfindung weist das medizinische Gerät Erfassungsmittel auf, mittels derer wenigstens eine Eigenschaft des oder der Disposables erfassbar ist.

Bei der Eigenschaft des Disposables kann es sich z.B. um farbige Markierungen, grafische Codes, wie z.B. Barcodes, oder-auch um Informationen handeln, die von - passiven oder aktiven Transponder an die Erfassungsmittel übertragen werden. Dies können beispielsweise auf der Umhüllung bzw. an dem Gehäuse des Disposables angebracht sein.

Denkbar ist es weiterhin, dass das Gerät über Vergleichsmittel verfügt, die ausgebildet sind, um die von den Erfassungsmitteln erfasste Information mit einer Sollinformation zu vergleichen. Auf diese Weise kann geräteseitig festgestellt werden, ob beispielsweise der richtige Lösungsbeutel in der richtigen Waagschale liegt, ob der richtige Filter in der richten Halterung angebracht ist und ob das richtige Schlauchset eingesetzt wurde, etc.

Um dem Nutzer eine Kontrollmöglichkeit zu verschaffen, kann das Gerät über Ausgebemittel verfügen, mittels derer die von den Erfassungsmitteln erfasste Information, das Ergebnis des in den Vergleichsmitteln vorgenommen Vergleichs oder eine darauf basierende Information für einen Nutzer des Gerätes erkennbar ausgebbar ist. So ist es möglich, dass in dem Gerät die Information hinterlegt ist, dass ein bestimmter Filter oder eine bestimmte Lösung verwendet werden soll. Wird festgestellt, dass dies nicht der Fall ist, kann eine entsprechende Meldung an den Nutzer ausgegeben und ggf. eine weitere Maßnahme ergriffen werden, wie beispielsweise die, dass die Inbetriebnahme des medizinischen Gerätes solange unterbleibt, bis der Fehler behoben ist.

So ist es möglich, dass das Gerät die tatsächliche Bestückung mit dem oder den Disposables überprüft und ggf. eine Rückmeldung über ein oder mehrere erkannte Disposables und/oder über Fehlkonfigurationen ausgibt.

Bei den Erfassungsmitteln kann es sich beispielsweise um eine Kamera, um Mittel zur Erkennung von RFID-Tags oder um sonstige Nahfelderfassungsmittel handeln, die auf Distanzen vorzugsweise von < 1m Kennzeichnungen des oder der Disposable erfassen können.

Die vorliegende Erfindung betrifft des Weiteren ein System umfassend wenigstens ein medizinisches Gerät nach einem der Ansprüche 1 bis 10, wobei das Gerät mit einem oder mehreren Disposables ausgestattet ist. Wie oben ausgeführt, kann es sich bei dem oder bei den Disposables beispielsweise um Lösungsbeutel, um Schlauchsets, um Filter, um Dialysatoren etc. handeln.

Vorzugsweise sind die die Disposables entsprechend des Behandlungsmodus farblich gestaltet. So ist es beispielsweise denkbar, dass im Rahmen einer Hämodiafiltrationsbehandlung eine der Waagschalen gelb beleuchtet wird und die andere Waagschale des Gerätes blau. Die auf die Waagschalen aufzulegen Lösungsbeutel, die einerseits Dialysat und andererseits Substituat enthalten, weisen eine entsprechende farbliche Gestaltung, also gemäß dem Beispiel ebenfalls gelb und blau auf.

Wird beispielsweise als Behandlungsmodus eine Hämodialysebehandlung gewählt, wird somit kein Substituat benötigt, sondern nur Dialysat, kann die Beleuchtung beider Waagschalen in Gelb erfolgen, so dass für den Nutzer sofort ersichtlich ist, dass auf beide Waagschalen mit Dialysat gefüllte Lösungsbeutel (mit einer gelben Farbcodierung) aufzulegen sind.

Die vorliegende Erfindung betrifft des Weiteren ein Verfahren mit den Merkmalen des Anspruchs 13.

Danach ist vorgesehen, dass die Leuchtmittel in Abhängigkeit von dem gewählten Behandlungsmodus des medizinischen Gerätes angesteuert werden, in dem das medizinische Gerät betrieben wird oder werden soll, wobei die Leuchtmittel derart angeordnet sind, dass das von diesen ausgehende Licht zumindest teilweise die eine oder mehreren funktionalen Einheiten beleuchtet.

Vorzugsweise wird das Licht wahlweise in unterschiedlichen Farben, Helligkeiten oder in unterschiedlichen zeitlichen Abläufen abgegeben.

Denkbar ist es, wenn eine Eigenschaft des oder der Disposables erfasst wird, wobei vorzugsweise vorgesehen ist, dass die von den Erfassungsmitteln erfasste Information mit einer Sollinformation verglichen wird.

In einer weiteren Ausgestaltung der Erfindung wird die von den Erfassungsmitteln erfasste Information, das Ergebnis des vorgenommen Vergleichs oder eine darauf basierende Information für einen Nutzer des Gerätes erkennbar ausgegeben, wie beispielsweise auf einem Bildschirm des Gerätes.

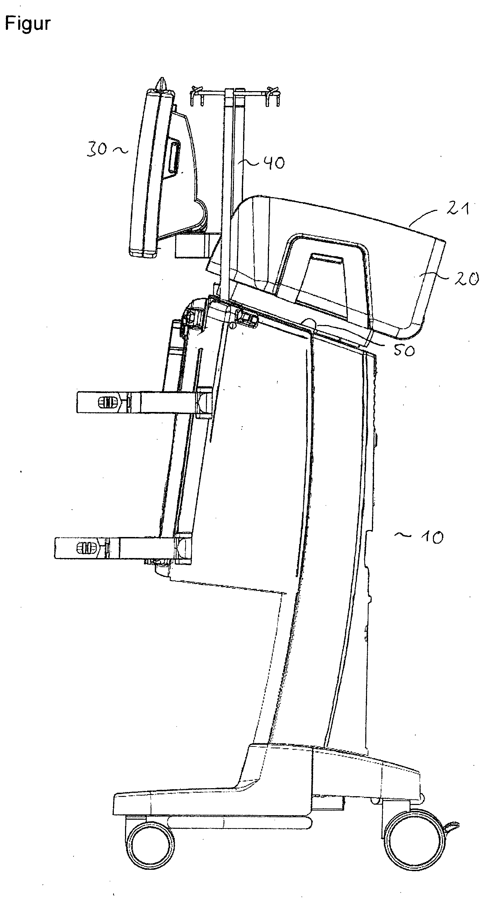

Weitere Einzelheiten und Vorteile der Erfindung werden anhand eines in der Zeichnung dargestellten Ausführungsbeispiels näher erläutert.

Die einzige Figur zeigt in schematischer Ansicht ein Dialysegerät gemäß der Erfindung.

Das Ausführungsbeispiel betrifft ein Dialysegerät 10, das in unterschiedlichen Behandlungsmodi verwendbar bzw. betreibbar ist.

Das Dialysegerät kann in einem ersten Behandlungsmodus für eine HDF, d.h. für eine Hämodiafiltration, und in einem zweiten Behandlungsmodus für eine HD, d.h. für eine Hämodialyse eingesetzt werden.

Eine weitere Unterscheidung der Behandlungsmodi kann im Hinblick auf das verwendete Antikoagulationsmittel vorgenommen werden (Calcium oder Heparin als Antikoagulationsmittel),

Das Gerät weist zwei Waagschalen 20, 21 auf, die aus einem transparenten Material ausgeführt sind, wie z.B. aus Kunststoff und die mit einer Wägezelle in Verbindung stehen, die permanent oder in bestimmten zeitlichen Abständen das Gewicht der in den Waagschalen befindlichen Lösungsbeuteln ermittelt.

Wie aus der Figur ersichtlich, befinden sich die Waagschalen 20, 21 oberhalb des Gerätekorpus und hinter dem Bildschirm 30, der die Schnittstelle zum Nutzer bildet und beispielsweise als Touchscreen ausgebildet, so dass Informationen ausgegeben und eingebbar sind. In dem Ausführungsbeispiel sind zwei nebeneinander angeordnete Waagschalen 20, 21 vorgesehen, von denen in der Seitenansicht gemäß der Figur nur (Waagschale 20) eine sichtbar ist. Die zweite Waagschale ist mit dem Pfeil und dem Bezugszeichen 21 angedeutet.

Das Bezugszeichen 40 kennzeichnet, einen Ständer zum Aufhängen von Lösungsbeuteln.

Unterhalb oder neben den Waagschalen 20, 21 befinden sich RGB LEDs, d.h. LEDs oder LED-Anordnungen, die wahlweise Licht in rot, grün oder blau oder auch in Mischfarben abgeben können. Diese sind ebenfalls nur schematisch wiedergegeben und mit dem Bezugszeichen 50 gekennzeichnet.

Das Gerät weist Wählmittel auf, mittels derer der Nutzer die Art der Behandlung (HDF oder HD) sowie das Antikoagulationsmittel (Citrat oder Heparin) einstellen kann. Die Wählmittel können beispielsweise Taster, Schalter oder auch Felder auf dem z.B. als Touchscreen ausgebildeten Bildschirm 30 sein.

Wird beispielsweise eine CVVHDF-Behandlung (CVVHDF = continuous venous-venous hemodiafiltration) durchgeführt mit einer Citrat-basierten Antikoagulation (Ci-Ca), werden mittels einer Steuereinheit die Leuchtmittel 50 derart angesteuert, dass die erste Waagschale in Gelb und die zweite Waagschale in blau beleuchtet werden.

Im Falle einer CVVHD-Behandlung (CWHD = continuous venous-venous hemodialysis) mit einer Citrat-basierten Antikoagulation, werden mittels einer Steuereinheit die Leuchtmittel 50 derart angesteuert, dass die erste Waagschale 20 in Gelb und die auch zweite Waagschale 21 in Gelb beleuchtet werden.

Wird eine CWHD-Therapie gewählt, bei der eine Heparin-Antikoagulation vorgenommen wird, kann auf eine Beleuchtung der ersten 20 und der zweiten Waagschale 21 auf blau umgestellt werden.

Die Beleuchtung der Waagschalen 20, 21 kann vor dem Beginn der Behandlung entsprechend gewählt werden, sowie auch bei einem Wechsel der Behandlung. In beiden Fällen ist für den Nutzer sofort ersichtlich, welchen farblich codierten Lösungsbeutel er in welche Waagschale 20, 21 zu legen hat.

Wird in dem o.g. Beispiel von einer Citrat basierten auf eine Heparin basierte Antikoagulation umgestellt (oder umgekehrt), so dass ein Wechsel der Lösungsbeutel erforderlich ist, erfolgt eine entsprechende Änderung der Beleuchtungsfarbe von gelb nach blau (oder umgekehrt).

Vorzugsweise entspricht die Beleuchtungsfarbe der Farbcodierung auf den Lösungsbeuteln, so dass für einen Nutzer sofort erkennbar ist, welcher Beutel auf welche Waagschale 20, 21 gelegt werden muss.

Um Fehlbehandlungen weitgehend auszuschließen, kann das Gerät mit Erfassungsmitteln ausgeführt sein, die ausgebildet sind, zu erkennen, welche Farbcodierung die Lösungsbeutel aufweisen und diese Information an Vergleichsmittel übertragen. Diese prüfen, ob die erfasste Farbcodierung einer Soll-Farbcodierung entspricht. Ist dies nicht der Fall, ist es denkbar, dass die Steuerungs- oder Regelungseinheit die Durchführung der Behandlung verhindert oder zumindest eine Wortmeldung an den Nutzer ausgibt, so dass dieser erkennt, dass eine falsche Zuordnung der Lösungsbeutel mit den Waagschalen 20, 21 erfolgt ist und ein Wechsel der Lösungsbeutel vorgenommen werden muss.

Sollen weiteren Lösungsbeutel eingeführt werden, kann die Farbcodierung der RGB LEDs oder sonstiger Leuchtmittel auf einfache Art und Weise erweitert werden.

## Patentansprüche

1. Medizinisches Gerät (10) mit einer oder mehreren funktionalen Einheiten, die ausgebildet sind, um im Betrieb des Gerätes mit einem oder mehreren Disposables zu interagieren, wobei das medizinische Gerät (10) des Weiteren ein oder mehrere Leuchtmittel (50) aufweist und wobei das Gerät in unterschiedlichen Behandlungsmodi betreibbar ist, **dadurch gekennzeichnet, dass** wenigstens eine Steuer- oder Regelungseinheit vorgesehen ist, die so ausgebildet sind, dass diese die Leuchtmittel (50) in Abhängigkeit von dem gewählten Behandlungsmodus des medizinischen Gerätes (10) ansteuert, in dem das medizinische Gerät betrieben wird oder werden soll, wobei die Leuchtmittel (50) derart angeordnet sind, dass das von diesen ausgehende Licht zumindest teilweise die eine oder mehreren funktionalen Einheiten beleuchtet, und wobei es sich bei der oder den funktionalen Einheiten um eine oder mehrere Waagschalen (20,21) zur Aufnahme von zu wiegenden Lösungsbeuteln handelt.

2. Medizinisches Gerät (10) nach Anspruch 1, **dadurch gekennzeichnet, dass** es sich bei dem medizinischen Gerät (10) um ein Dialysegerät handelt.

3. Medizinisches Gerät (10) nach einem der vorhergehenden Ansprüche, **dadurch gekennzeichnet, dass** es sich bei der oder den funktionalen Einheiten um eine oder mehrere Halterungen oder Aufnahmen für ein oder mehrere Schlauchsets, Filter oder Dialysatoren handelt.

4. Medizinisches Gerät (10) nach einem der vorhergehenden Ansprüche, **dadurch gekennzeichnet, dass** die wenigstens eine funktionale Einheit teilweise oder vollständig transparent oder transluzent ausgebildet ist.

5. Medizinisches Gerät (10) nach einem der vorhergehenden Ansprüche, **dadurch gekennzeichnet, dass** die Leuchtmittel (50) derart ausgebildet sind, dass sie Licht wahlweise in unterschiedlichen Farben, Helligkeiten oder in unterschiedlichen zeitlichen Abläufen abgeben.

6. Medizinisches Gerät (10) nach einem der vorhergehenden Ansprüche, **dadurch gekennzeichnet, dass** die Leuchtmittel als LEDs, insbesondere als RGB-LEDs ausgebildet sind oder LEDs und insbesondere RGB-LEDs umfassen.

7. Medizinisches Gerät (10) nach einem der vorhergehenden Ansprüche, **dadurch gekennzeichnet, dass** das medizinische Gerät Erfassungsmittel aufweist, mittels dessen wenigstens eine Eigenschaft des oder der Disposables erfassbar ist.

8. Medizinisches Gerät (10) nach Anspruch 8, **dadurch gekennzeichnet, dass** das medizinische Gerät (10) über Vergleichsmittel verfügt, die ausgebildet sind, um die von den Erfassungsmitteln erfasste Information mit einer Sollinformation zu vergleichen.

9. Medizinisches Gerät (10) nach Anspruch 7 und 8, **dadurch gekennzeichnet, dass** das medizinische Gerät über Ausgebemittel (30) verfügt, mittels derer die von den Erfassungsmitteln erfasste Information, das Ergebnis des in den Vergleichsmitteln vorgenommen Vergleichs oder eine darauf basierende Information für einen Nutzer des Gerätes (10) erkennbar ausgebbar ist.

10. Medizinische Gerät (10) nach einem der Ansprüche 7 bis 9, **dadurch gekennzeichnet, dass** es sich bei den Erfassungsmitteln um eine oder mehrere Kameras, Mittel zur Erkennung von RFID-Tags oder um sonstige Nahfelderfassungsmittel handelt.

11. System umfassend wenigstens ein medizinisches Gerät (10) nach einem der Ansprüche 1 bis 10, wobei das medizinische Gerät (10) mit einem oder mehreren Disposables ausgestattet ist.

12. System nach Anspruch 11, **dadurch gekennzeichnet, dass** das oder die Disposables entsprechend des Behandlungsmodus farblich gestaltet sind.

13. Nicht-therapeutisches und nicht-chirurgisches Verfahren zum Betreiben eines medizinischen Gerätes (10) wobei das medizinische Gerät (10) eine oder mehrere funktionalen Einheiten aufweist, die ausgebildet sind, um im Betrieb des Gerätes mit einem oder mehreren Disposables zu interagieren, wobei das medizinische Gerät (10) des Weiteren ein oder mehrere Leuchtmittel (50) aufweist und wobei das Gerät (10) in unterschiedlichen Behandlungsmodi betreibbar ist, **dadurch gekennzeichnet, dass** die Leuchtmittel (50) in Abhängigkeit von dem gewählten Behandlungsmodus des medizinischen Gerätes (10) angesteuert werden, in dem das medizinische Gerät (10) betrieben wird oder werden soll, wobei die Leuchtmittel (50) derart angeordnet sind, dass das von diesen ausgehende Licht zumindest teilweise die eine oder mehreren funktionalen Einheiten beleuchtet und wobei es sich bei der oder den funktionalen Einheiten um eine oder mehrere Waagschalen zur Aufnahme von zu wiegenden Lösungsbeuteln handelt.

14. Verfahren nach Anspruch 13, **dadurch gekennzeichnet, dass** das Licht wahlweise in unterschiedlichen Farben, Helligkeiten oder in unterschiedlichen zeitlichen Abläufen abgegeben wird.

15. Verfahren nach Anspruch 13 oder 14, **dadurch gekennzeichnet, dass** wenigstens eine Eigenschaft des oder der Disposables erfasst wird, wobei vorzugsweise vorgesehen ist, dass die von den Erfassungsmitteln erfasste Information mit einer Sollinformation verglichen wird.

16. Verfahren nach Anspruch 15, **dadurch gekennzeichnet, dass** die von den Erfassungsmitteln erfasste Information, das Ergebnis des vorgenommen Vergleichs oder eine darauf basierende Information für einen Nutzer des Gerätes (10) erkennbar ausgegeben wird.

## Claims

1. Medical device (10) having one or more functional units which are designed to interact with one or more disposables during operation of the device, the medical device (10) further having one or more illuminating means (50), and the device being operable in different treatment modes, **characterized in that** at least one control or regulating unit is provided, which is designed in such a way that it controls the illuminating means (50) in dependence on the selected treatment mode of the medical device (10) in which the medical device is or is to be operated, wherein the illuminating means (50) are arranged in such a way that the light emanating therefrom at least partially illuminates the one or more functional units, and wherein the functional unit or units are one or more scale pans (20, 21) for receiving solution bags to be weighed.

2. Medical device (10) according to claim 1, **characterized in that** the medical device (10) is a dialysis device.

3. Medical device (10) according to any one of the preceding claims, **characterized in that** the functional unit or units are one or more holders or receptacles for one or more tube sets, filters or dialyzers.

4. Medical device (10) according to any one of the preceding claims, **characterized in that** the at least one functional unit is partially or completely transparent or translucent.

5. Medical device (10) according to any one of the preceding claims, **characterized in that** the illuminating means (50) are configured to selectively emit light in different colors, brightnesses or in different temporal sequences.

6. Medical device (10) according to one of the preceding claims, **characterized in that** the illuminating means are designed as LEDs, in particular as RGB LEDs, or comprise LEDs and in particular RGB LEDs.

7. Medical device (10) according to one of the preceding claims, **characterized in that** the medical device comprises detection means by means of which at least one property of the disposable or disposables is detectable.

8. Medical device (10) according to claim 7, **characterized in that** the medical device (10) comprises comparison means adapted to compare the information detected by the detection means with a target information.

9. Medical device (10) according to claim 7 and 8, **characterized in that** the medical device has output means (30) by means of which the information detected by the detection means, the result of the comparison made in the comparison means or information based thereon can be output recognizably for a user of the device (10).

10. Medical device (10) according to any one of claims 7 to 9, **characterized in that** the detection means are one or more cameras, RFID tag detection means or other near field detection means.

11. A system comprising at least one medical device (10) according to any one of claims 1 to 10, wherein the medical device (10) is provided with one or more disposables.

12. System according to claim 11, **characterized in that** the disposable or disposables are colored according to the treatment mode.

13. A non-therapeutic and non-surgical method of operating a medical device (10), the medical device (10) comprising one or more functional units adapted to interact with one or more disposables during operation of the device, wherein the medical device (10) further comprises one or more illuminating means (50), and wherein the device (10) is operable in different treatment modes, **characterised in that** the illuminating means (50) are controlled in dependence on the treatment mode in which the medical device (10) is or is to be operated, that the illuminating means (50) are controlled in dependence on the selected treatment mode of the medical device (10) in which the medical device (10) is or is to be operated, wherein the illuminating means (50) are arranged such that the light emanating therefrom at least partially illuminates the one or more functional units and wherein the one or more functional units are one or more scale pans for receiving solution bags to be weighed.

14. Method according to claim 13, **characterized in that** the light is optionally emitted in different colors, brightnesses or in different temporal sequences.

15. Method according to claim 13 or 14, **characterized in that** at least one property of the disposable or disposables is detected, preferably providing that the information detected by the detection means is compared with a target information.

16. Method according to claim 15, **characterized in that** the information detected by the detection means, the result of the comparison made or information based thereon is output recognizably for a user of the device (10).

## Revendications

1. Appareil médical (10) avec une ou plusieurs unités fonctionnelles qui sont configurées pour interagir avec un ou plusieurs composants jetables lors de l'exploitation de l'appareil, l'appareil médical (10) présentant en outre un ou plusieurs moyens d'éclairage (50) et l'appareil pouvant être exploité dans différents modes de traitement, **caractérisé en ce qu'**au moins une unité de commande ou de régulation est prévue, qui est configurée de telle sorte que celle-ci commande les moyens d'éclairage (50) en fonction du mode de traitement sélectionné de l'appareil médical (10) dans lequel l'appareil médical est ou doit être exploité, les moyens d'éclairage (50) étant agencés de telle sorte que la lumière émise par ceux-ci éclaire au moins partiellement les une ou plusieurs unités fonctionnelles, et la ou les unités fonctionnelles consistant en un ou plusieurs plateaux de balance (20, 21) destinés à recevoir des sachets de solution à peser.

2. Appareil médical (10) selon la revendication 1, **caractérisé en ce que** l'appareil médical (10) est un appareil de dialyse.

3. Appareil médical (10) selon l'une quelconque des revendications précédentes, **caractérisé en ce que** la ou les unités fonctionnelles sont un ou plusieurs supports ou logements pour un ou plusieurs ensembles de tubes, filtres ou dialyseurs.

4. Appareil médical (10) selon l'une quelconque des revendications précédentes, **caractérisé en ce que** l'au moins une unité fonctionnelle est configurée sous forme partiellement ou entièrement transparente ou translucide.

5. Appareil médical (10) selon l'une quelconque des revendications précédentes, **caractérisé en ce que** les moyens d'éclairage (50) sont configurés de telle sorte qu'ils émettent de la lumière sélectivement dans différentes couleurs, luminosités ou selon différentes séquences temporelles.

6. Appareil médical (10) selon l'une quelconque des revendications précédentes, **caractérisé en ce que** les moyens d'éclairage sont configurés sous forme de LED, en particulier sous forme de LED RVB, ou comprennent des LED et en particulier des LED RVB.

7. Appareil médical (10) selon l'une quelconque des revendications précédentes, **caractérisé en ce que** l'appareil médical présente des moyens de détection au moyen desquels au moins une propriété du ou des composants jetables peut être détectée.

8. Appareil médical (10) selon la revendication 8, **caractérisé en ce que** l'appareil médical (10) dispose de moyens de comparaison qui sont configurés pour comparer l'information détectée par les moyens de détection à une information de consigne.

9. Appareil médical (10) selon les revendications 7 et 8, **caractérisé en ce que** l'appareil médical dispose de moyens de délivrance (30) au moyen desquels l'information détectée par les moyens de détection, le résultat de la comparaison effectuée dans les moyens de comparaison ou une information basée sur celui-ci peut être délivré de manière reconnaissable pour un utilisateur de l'appareil (10).

10. Appareil médical (10) selon l'une quelconque des revendications 7 à 9, **caractérisé en ce que** les moyens de détection est une ou plusieurs caméras, moyens de reconnaissance d'étiquettes RFID ou autres moyens de détection en champ proche.

11. Système comprenant au moins un appareil médical (10) selon l'une quelconque des revendications 1 à 10, dans lequel l'appareil médical (10) est équipé d'un ou de plusieurs composants jetables.

12. Système selon la revendication 11, **caractérisé en ce que** le ou les composants jetables sont conçus sous forme colorée en fonction du mode de traitement.

13. Procédé non thérapeutique et non chirurgical d'exploitation d'un appareil médical (10), l'appareil médical (10) présentant une ou plusieurs unités fonctionnelles qui sont configurées pour interagir avec un ou plusieurs composants jetables lors de l'exploitation de l'appareil, l'appareil médical (10) présentant en outre un ou plusieurs moyens d'éclairage (50) et l'appareil (10) pouvant être exploité dans différents modes de traitement, **caractérisé en ce que** les moyens d'éclairage (50) sont commandés en fonction du mode de traitement sélectionné de l'appareil médical (10) dans lequel l'appareil médical (10) est ou doit être exploité, les moyens d'éclairage (50) étant agencés de telle sorte que la lumière émise par ceux-ci éclaire au moins partiellement les une ou plusieurs unités fonctionnelles, et la ou les unités fonctionnelles sont un ou plusieurs plateaux de balance destinés à recevoir des sachets de solution à peser.

14. Procédé selon la revendication 13, **caractérisé en ce que** la lumière est émise sélectivement en différentes couleurs, luminosités ou selon différentes séquences temporelles.

15. Procédé selon la revendication 13 ou 14, **caractérisé en ce qu'**au moins une caractéristique du ou des composants jetables est détectée, il étant de préférence prévu que l'information détectée par les moyens de détection est comparée à une information de consigne.

16. Procédé selon la revendication 15, **caractérisé en ce que** l'information détectée par les moyens de détection, le résultat de la comparaison effectuée ou une information basée sur celui-ci est délivré de manière reconnaissable pour un utilisateur de l'appareil (10).
